# EUROPEAN PATENT SPECIFICATION

(11) **EP 2 430 430 B1**
(45) Date of publication and mention of the grant of the patent: **12.02.2020**
(21) Application number: 10722227.5
(22) Date of filing: 10.05.2010
(51) Int. Cl.: G01N 21/65, G01N 33/22, G01N 21/03

(54) **METHOD FOR REDUCING LOSS OF ELECTROMAGNETIC RADIATION IN DETECTION APPLICATIONS**
VERFAHREN ZUR VERRINGERUNG ELEKTROMAGNETISCHER STRAHLUNG BEI NACHWEISANWENDUNGEN
PROCÉDÉ POUR RÉDUIRE LES PERTES DE RAYONNEMENT ÉLECTROMAGNÉTIQUE DANS DES APPLICATIONS DE DÉTECTION

(30) Priority: 11.05.2009 US 177178 P
(43) Date of publication of application: 21.03.2012
(73) Proprietor: Smiths Detection Inc., Danbury, CT 06810 (US)
(72) Inventor: ZOU, Peng, Brookfield CT 06804 (US); LEVY, Dustin, Danbury CT 06810 (US)
(74) Representative: Leach, Sean Adam
(86) International application number: PCT/US2010/034230
(87) International publication number: WO 2010/132354

(56) References cited:
- WO-A1-01/36949
- WO-A2-2005/012553
- JP-A- 55 050 144
- US-A- 5 823 951
- US-A1- 2008 186 471
- COATES JOHN: "Molecular spectroscopy workbench new technologies for process analytical andquality control applications: Compact Raman" SPECTROSCOPY, ADVANSTAR COMMUNICATIONS, US, vol. 21, no. 2, 1 February 2006 (2006-02-01), pages 68-74, XP009096137 ISSN: 0887-6703

## Description

This application claims benefit to U.S. provisional patent application no. 61/177,178, filed May 11, 2009 to Zou et al.*.*

This application provides methods for improving the identification and characterization of analytes in containers with rough surfaced materials, such as frosted glass, using radiation and wave-based techniques.

### BACKGROUND

Maintaining and improving the security of our transportation networks, ports and borders, remains an important objective in view of continued terrorist threats. A number of techniques have been employed to detect dangerous or illicit articles, such as narcotics, explosives, chemical warfare agents, and biological toxins. For example, screening methods for dangerous and illicit substances include X-ray, computed tomography ("CT"), magnetic resonance, ion mobility spectroscopy, and polymerase chain reaction (PCR).

Radiation-based and wave-based detection are some techniques that have been employed to screen luggage and other articles. These techniques are advantageous, because, among other things, they do not require destructive or intrusive testing. Examples of these techniques for detection of analytes include Raman spectroscopy, infrared, near infrared, millimeter wave, tetrahertz, visible, UV, VUV, and ultrasound techniques.

Raman spectroscopy can be used to screen for, among other things, liquid explosives. This is an application of considerable importance, because terrorists have already attempted to use liquid explosives, such as the London attacks of July 2005 and failed bombings of transatlantic flights in 2006. Existing noninvasive screening methods have significant limitations in detecting liquid explosives making Raman spectroscopy an attractive alternative.

Raman spectroscopy has potential to be a very valuable tool for screening articles. Because it works based on transmission and detection of light, technicians do not necessarily need to come into contact with the unknown substance or remove unknown substance from its container. For example, a container purportedly containing milk for a child boarding an airplane does not need to be opened and a sample removed, if Raman spectroscopy is used to determine the contents of the container. This reduces the risk of contamination, both for the technician and for the substance, and can speed screening. Moreover, Raman spectroscopy can determine the identity of a substance, or verify that a substance is not one of a number of substances of interest, relatively quickly. Raman spectroscopy also can be deployed in a handheld, battery-powered apparatus. Accordingly, Raman spectroscopy can be a valuable tool in screening articles for dangerous or illicit substances.

Radiation and wave-based techniques, such as Raman spectroscopy, however, also currently suffer from a number of limitations. For example, the effectiveness of these techniques can be reduced, or even eliminated, when a rough, discontinuous, and/or diffuse surface, such as frosted glass or plastic, separates the instrument from the analyte or article of interest. The rough surface can cause a loss of electromagnetic radiation (EMR) by scattering, dispersion, and/or diffraction at the surface. In the case of Raman spectroscopy, this problem can manifest itself in an inability to detect analytes in containers with rough surfaces.

Efforts have been made to overcome the limitations of Raman spectroscopy to allow its use to detect explosives in commonly used containers. For example, Eliasson et al., ANAL. CHEM. 79:8185-89 (2007) uses spatially offset Raman spectroscopy (SORS) to detect potentially explosive solutions in small white containers. But SORS requires more optical components that traditional backscattering Raman spectroscopy and, therefore, is less compact that traditional backscattering Raman instruments.

Thus, a need exists to reduce the loss of EMR caused by rough surfaces when using radiation and wave-based analysis techniques.

Coates, John, "Molecular Spectroscopy Workbench, New Technologies for Process Analytical and Quality Control Applications: Compact Raman, Spectrosopy 21(2), February 2006, pages 68-74, ISSN: 0887-6703 discloses process analytical and quality control applications of molecular spectroscopy.

WO 2005/012533-A2 discloses a method and system for non-invasive, in vivo optical detection of analytes.

### SUMMARY

The present application provides methods for performing radiation and wavebased optical techniques. These methods reduce the amount of electromagnetic radiation loss caused by rough surfaces by employing a surface layer to the rough surfaces. In the case of Raman spectroscopy, these methods can reduce scattering, dispersion, or diffraction of light caused by a rough surface.

According to the invention, the application provides a method of reducing the loss of electromagnetic radiation during Raman spectroscopy wherein the loss is caused by passing the electromagnetic radiation through material comprising glass or plastic with a rough surface, the method comprising applying a surface layer to the rough surface wherein the material is in the form of a container before electromagnetic radiation is passed through the material, wherein the surface layer decreases the loss of electromagnetic radiation resulting from passing the electromagnetic radiation through the material, and passing electromagnetic radiation through the container with the surface layer in order to identify an analyte arranged in the container, wherein the surface layer is selected to have optical properties that do not negatively interfere with analysis of the analyte, the analyte being one of an explosive or a narcotic.

Also described herein is an embodiment of performing Raman spectroscopy on an analyte in a container having a rough surface, comprising: applying a surface layer to the rough surface; illuminating the analyte with monochromatic light by passing monochromatic light through the rough surface of the container; and detecting Raman scattering caused by the analyte using a detector, wherein the surface layer reduces the loss of electromagnetic energy resulting from (i) passing the monochromatic light through the rough surface to the analyte or (ii) the photons passing through the rough surface to the detector.

In another method according to the invention, there is provided a method of reducing the loss of electromagnetic radiation caused by passing electromagnetic radiation through material with a rough surface, comprising applying a surface layer to the rough surface of a material in the form of a substrate before electromagnetic radiation is passed through the material, wherein the surface is rough due to pits or gaps caused by at least one of: molding, chemical etching, or sandblasting; wherein the surface layer decreases the loss of electromagnetic radiation resulting from passing the electromagnetic radiation through the material, wherein the substrate is the optical head of an analysis instrument.

### BRIEF DESCRIPTION OF THE FIGURES

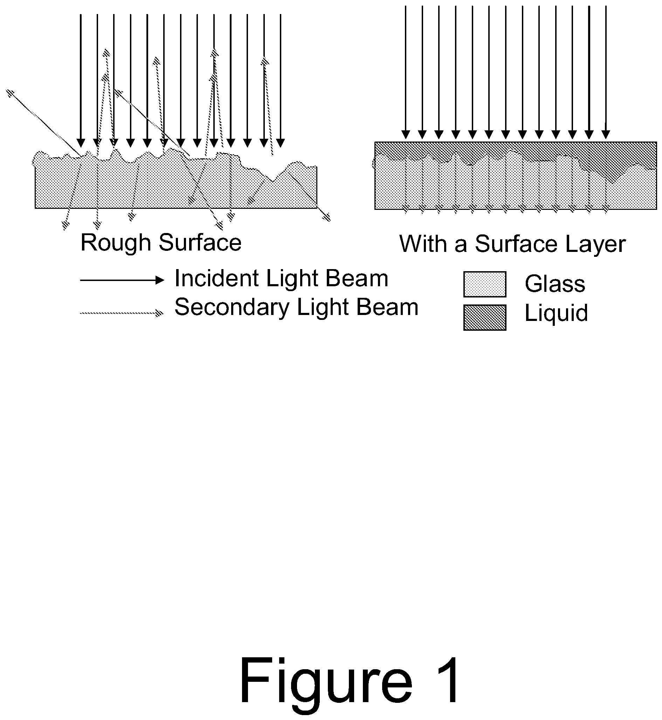

Figure 1 is a schematic illustration of how a substance, such as a liquid, can be applied to a rough surface to fill pits, gaps, and other surface irregularities to improve optical transparency.

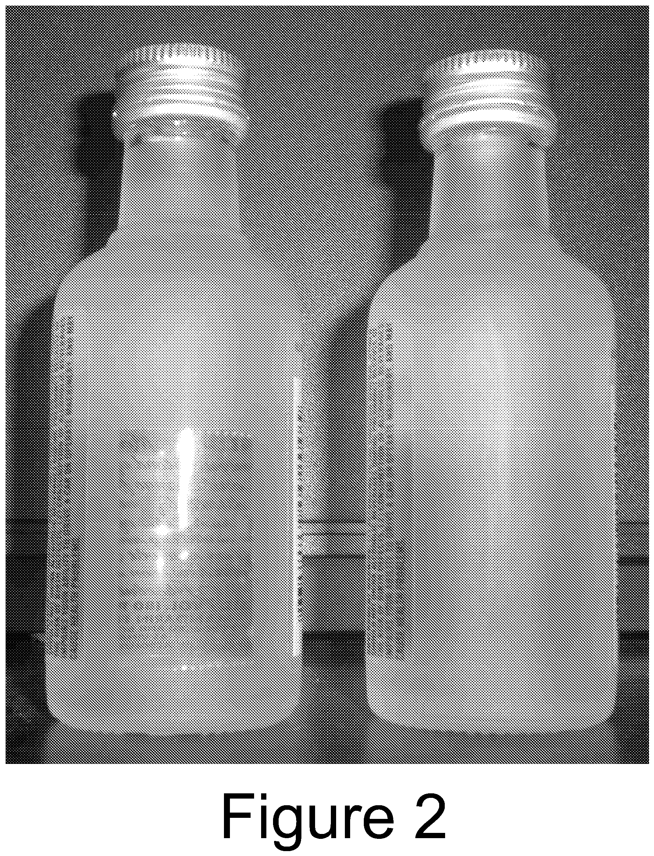

Figure 2 shows two frosted glass bottles. A surface smoothing layer, adhesive tape, has been applied to the bottle on the left. The application of the adhesive tape improves optical transparency as evidenced by the ability to read the text on the opposite side of the bottle.
Figure 3 shows the effects of applying a surface layer in the form of a transparent adhesive tape to a frosted glass bottle. Figure 3(a) shows the spectrum obtained without the surface layer, and Figure 3(b) shows the spectrum obtained with a surface layer. The surface layer greatly increased the strength of the signal from the analyte, an organic ketone.

### DETAILED DESCRIPTION

The present application provides methods for performing radiation and wave-based optical techniques that reduce the electromagnetic radiation (EMR) loss that occurs due to the presence of rough surfaces. Specifically, the presence of rough surfaces can cause loss of EMR through a variety of mechanisms, such as scatter, reflection, diffraction, and dispersion. The loss of EMR, in turn, can reduce the effectiveness of radiation and wave-based detection techniques due to loss of signal.

Applicants surprisingly discovered that the loss of EMR caused by rough surfaces can be reduced or eliminated by applying a surface layer to the rough surface. The surface layer reduces loss of EMR by filling or masking pits, gaps, and other irregular features in order to achieve a surface more transparent to the optical energy or waves being employed. The type of surface material is not limited, as described in detail below, and can be any suitable material, such as, for example, a film, such as adhesive tape and plastic film, or a liquid. In the case of Raman spectroscopy, application of surface layers to frosted glass bottles significantly increases sensitivity of detection methods.

Unless explicitly stated otherwise, "and" can mean "or," and "or" can mean "and." For example, if a feature is described as having A, B, or C, the feature can have A, B, and C, or any combination of A, B, and C. Similarly, if a feature is described as having A, B, and C, the feature can have only one or two of A, B, or C.

Unless explicitly stated otherwise, "a" and "an" can mean "one or more than one." For example, if a device is described as having a feature X, the device may have one or more of feature X.

"Radiation-based" and "wave-based" techniques refer to techniques that rely on the transmission and/or detection of radiation or wave energy, respectively, to identify or characterize an analyte or article of interest. There can be overlap between radiation-based and wave-based techniques. Examples of radiation-based techniques include, but are not limited to, optical techniques, such as Raman spectroscopy, infrared (dispersive and non-dispersive, including, for example, FTIR), visible and ultraviolet spectroscopy, laser induced chemical excitation techniques, and microwave spectroscopy . Examples of wave-based techniques include, but are not limited to, ultrasonic spectroscopy, acoustic spectroscopy, millimeter wave scanning, and terahertz scanning.

"Raman spectroscopy" includes any spectroscopy technique that uses Raman scattering to identify or characterize substances. Examples of specific Raman spectroscopy techniques include, but are not limited to, Raman backscattering spectroscopy, Surface Enhanced Raman Spectroscopy (SERS), Resonance Raman spectroscopy, Surface Enhanced Resonance Raman Spectroscopy (SERRS), Hyper Raman, Spontaneous Raman Spectroscopy, Stimulated Raman Spectroscopy, Spatially Offset Raman Spectroscopy (SORS), Coherent anti-Stokes Raman spectroscopy (CARS), Raman optical activity (ROA), Transmission Raman, Inverse Raman spectroscopy, and Tip-Enhanced Raman Spectroscopy (TERS). "Monochromatic light" refers to light of uniform wavelength and can be generated using a laser, for example. Monochromatic light can consist of light of a single wavelength or light in a narrow range of wavelengths. Monochromatic light can include light in the visible, near infrared, or near ultraviolet ranges.

"Analyte" refers to any substance to be identified or analyzed. Analytes include, but are not limited to, explosives, narcotics, drugs, chemicals, toxic industrial chemicals, chemical warfare agents, and biological toxins. In some embodiments, the analyte is a peroxide-containing solution, including, for example, hydrogen peroxide or other peroxides.

"Container" refers to any vessel that can hold an analyte. Examples of containers include, but are not limited to, bottles, capsules, flasks, jars, storage trays, vials, dispensers, spray bottles, pump bottles, misters, flasks, pill bottles, pill boxes, pouches, and flip-top bottles. Containers can be made of any suitable material, such as, for example, plastic and glass. Containers can be transparent, translucent, or opaque to visible light. Containers can be made from a variety of materials with differing properties. For example, a portion of a container can be opaque and other portions transparent or translucent.

"Frosted glass" and "frosted plastic" includes frosted glass and frosted plastic made by any method, including acid etching and sand-blasting.

"Electromagnetic radiation," also referred to as "EMR," refers to any type of electromagnetic radiation. Examples of electromagnetic radiation include, but are not limited to, radio waves, microwaves, terahertz radiation, infrared radiation, light, X-rays and gamma rays. Light includes, but is not limited to, visible light, ultraviolet light, and infrared light.

"Surface layer" refers to any material that can be applied to a surface to reduce the loss of EMR caused by passing optical or wave energy through the surface. In some embodiments, the surface layer serves one or more of the following functions or has one or more of the following characteristics:
1. smoothing irregularities by filling the rough surface pits, or gaps;
2. masking irregularities by filling the rough surface pits, or gaps;
3. a refraction index similar to that of the surface; and
4. optical properties that do not negatively interfere with the analysis.

The "surface layer" can be a liquid, solid or gel. Examples of surface layer materials include, but are not limited to adhesive tape, gels, epoxies, or polymer compounds, liquids (including water), polyester sheets, (including polyethylene terephthalate sheets such as MYLAR® available from Dupont Teijin Films, Hopewell, Virginia), vinyl films, synthetic and natural wax products, p vinyl films, parafilm, lacquers, and varnishes. In some embodiments, the polymers can be either organic or inorganic. In some embodiments, the surface layer can be applied as a film, such as a polymer film. In some embodiments, the substance can be optically transparent or translucent to light, including monochromatic light. In some embodiments, the surface layer can be readily removed from the container surface without using special tools or solvents. In other embodiments, the surface layer is not easily removed or permanently alters the surface.

The degree to which the surface layer decreases the loss of electromagnetic radiation resulting from passing the electromagnetic radiation through the material will vary depending on the specific application. As compared to passing EMR through a rough surface without a surface layer, passing the EMR through a rough surface with a surface layer can increase the amount of EMR transmitted by 10%, 25%, 50%, 75%, 85%, 95%, 100%, 200%, 300%, or 500% or more. In some embodiments, the increase in EMR from using a surface layer is an order of magnitude, two orders of magnitude, three orders of magnitude, four orders of magnitude, or more. The benefit of using a surface layer can also be quantified by comparing the relevant signal obtained when using a surface layer to signal strength when no surface layer is used. For example, the strength of the Raman backscattering signal can be used to quantify the effects in a Raman spectroscopy application. In some embodiments, use of a surface layer increase signal strength by 10%, 25%, 50%, 75%, 85%, 95%, 100%, 2005, 300%, or 500% or more. In some embodiments, the increase in signal strength from using a surface layer is an order of magnitude, two orders of magnitude, three orders of magnitude, four orders of magnitude, or more.

"Substrate" refers to the material to which the surface layer is applied. In some embodiments, the substrate has a rough surface. The surface can be rough due to pits or gaps. The roughness can be caused by molding, chemical etching, or sandblasting, for example. In some embodiments, the substrate can be the optical head of the analysis instrument. In these embodiments, the optical head itself can be a rough surface or the optical head can make contact with the rough surface during the analysis such that the surface layer on the optical head contacts the rough surface. In some embodiments, the substrate will be a container or a portion of a container.

The surface layer can be applied to the rough surface of the substrate using any suitable means. For example, the surface layer can be applied by spraying the rough surface with a material that forms the surface layer. The material can form the surface area upon contacting the surface or may need to be dried or cured. The drying or curing can result simply from the passage or time or can be facilitated, such as by the application of heat or ultraviolet radiation.

In some embodiments, the surface layer can be applied using an adhesive. For example, a plastic film can be applied to a rough surface using adhesive applied to either the rough surface or the plastic film. Any suitable adhesive can be used. Any suitable method can be used to apply adhesive, including spraying the adhesive or brushing the adhesive. Commonly available transparent adhesive tape can form a surface layer, and these transparent adhesive tapes can by applied by simply pressing them against the rough surface. Alternatively, an adhesive material can be applied to the rough surface, and a patch, such as a transparent patch, applied to the adhesive on the rough surface. The patch can be preformed or can be formed to fit a particular surface.

In some embodiments, the surface layer can be applied by submerging the rough surface in a liquid or gel that forms the surface layer. The liquid itself can constitute the surface layer or may contain substances that form the surface layer. A portion of an apparatus that either generates or detects electromagnetic radiation can be submerged with the rough surface, but in some embodiments, only the rough surface is submerged. In some embodiments, the surface layer or apparatus portion is removed from the liquid before the analysis commences. In these embodiments, the liquid, or a component within the liquid, is retained on the surface or apparatus portion to form a surface layer.

In some embodiments, more than one surface layer can be employed. The surface layers can be the same or different types of materials. For example, a first surface layer can be liquid or gel, and a second surface layer can be a solid, such as a plastic film overlying the first surface layer. In some embodiments, multiple surface layers are employed where the softest or most deformable surface layer is closest the rough surface and one or more increasing hard or less deformable surface layers are progressively applied on top of the first surface layer. One of skill in the art could readily select combinations of surface layers and their relative positions based on the particular application.

In some embodiments, the surface layer is reusable. In some embodiments, the surface layer can be used for more than one round or type of analysis. In some embodiments, the surface layer can be removed from a surface after analysis and placed on a different surface for another analysis using the same or different analysis technique. Examples of surface layers that can be reusable include, but are not limited to, liquids and films, such as transparent adhesive tapes.

In some embodiments, one or more protective layers are applied over the surface layer or is applied to the surface before application of the protective layer. The protective layer can serve one or more of the following functions: (1) protecting the surface layer before or during analysis; (2) preventing the analysis instrument from contacting the surface layer; (3) protecting technicians or others from contacting the surface layer; or (4) facilitating removal of the surface layer. The protective layer can be formed from any suitable substance including, but not limited to, films, plastics, lacquers, or varnishes. The protective layer can be a solid, liquid, or gel. Any suitable method of applying the protective layer can be employed.

In some embodiments, the surface layer is not applied directly to the surface. Rather, one or more other materials or layers can separate the surface layer from the surface. For example, an adhesive can separate the surface layer from the surface. As another example, a protective layer can separate the surface layer from the surface.

In one particular application, surface layers can be employed to facilitate Raman spectroscopy. Raman spectroscopy can be employed to determine the identity of an analyte in a container. The container can be a bottle possessed by a passenger boarding an aircraft, for example. The surface layer can be applied to the container before being subjected to Raman spectroscopy to reduce the loss of EMR, such as the diffraction of monochromatic light due to the surface of the container or loss of Raman scattering signal by photons passing back through the surface. The use of surface layers in Raman spectroscopy will be particularly advantageous when the container is made of a particularly rough material, such as frosted glass or frosted plastic.

Surface layers can be applied in the field where a portable device might be employed. In some embodiments, a Raman spectroscopy technician can apply a transparent adhesive tape to a container containing an analyte to be subjected to Raman spectroscopy. In other embodiments, the technician can create a surface layer by submerging the container in a liquid, such as water. In yet another embodiment, the technician applies a surface layer by spraying a material onto the container. In some embodiments, the surface layer applied by the technician can be easily removed such that no special tools or solvents are required.

In some embodiments, the surface layer will be applied to only the surface of a container holding the analyte. In some embodiments, the surface layer can be applied to the optical head of the instrument. In other embodiments, however, a surface layer can be applied to both the optical head of the instrument and to the surface of the container. This can be achieved in a number of ways, including the ways described herein. For example, different surface layers can be applied to the optical head and the container. As another example, the optical head and the container can both be submerged in a liquid to form a surface layer.

While the application of surface layers has been described primarily in the context of Raman spectroscopy, surface layers can be readily applied to any technique that depends on the transmission of EMR through a material surface or detection of EMR passing through a material surface.

### Example 1: Raman Spectroscopy Using A Surface Layer

The effectiveness of surface layers was tested by comparing Raman spectroscopy detection of a particular organic ketone compound, which will simply be referred to as "ketone," in frosted glass containers as compared to Raman spectroscopy detection of ketone in frosted glass containers with different surface layers. A RespondeR RCI (Raman Chemical Identifier, Smiths Detection, Watford, England) was used to perform the Raman spectroscopy. Experimental conditions were identical except for the focus, presence of surface layer, and type of surface layer, as described in Table 1. The results of the testing are reported in Table 1. The "Quality" value is a dimensionless number. The higher the quality, the better the match between spectrum of a standard and spectrum of an unknown chemical. Higher quality typically indicate spectrum of the unknown has better S/N ratio.

| **TABLE 1** | | | |
|---|---|---|---|
| **Analyte** | **Container** | **Focus¹** | **Quality²** |
| ketone | Frosted Glass Bare | near | Miss |
| ketone | Frosted Glass Bare | near | 0.0561 |
| ketone | Frosted Glass Bare | near | 0.3831 |
| ketone | Frosted Glass Bare | middle | miss |
| ketone | Frosted Glass Bare | middle | 0.3075 |
| ketone | Frosted Glass Bare | middle | 0.2124 |
| ketone | Frosted Glass Bare | far | miss |
| ketone | Frosted Glass Bare | far | 0.2496 |
| ketone | Frosted Glass and Spectrometer in water | near | 0.4896 |
| ketone | Frosted Glass in beaker with water | near | 0.4155 |
| ketone | Frosted Glass in plastic bag in water | near | 0.6105 |
| ketone | Frosted Glass in plastic bag in water | near | 0.6366 |
| ketone | Frosted Glass in plastic bag in water | near | 0.614 |
| ketone | Frosted Glass in plastic bag in water | near | 0.6144 |
| ketone | Frosted Glass in plastic bag in water | middle | 0.5936 |
| ketone | Frosted Glass in plastic bag in water | middle | 0.6938 |
| ketone | Frosted Glass in plastic bag in water | middle | 0.7389 |
| ketone | Frosted Glass in plastic bag in water | far | 0.6393 |
| ketone | Frosted Glass in plastic bag in water | far | 0.6227 |
| ketone | Frosted Glass in plastic bag in water | far | 0.6399 |
| ketone | Frosted Glass in plastic patch with water | Near | 0.5733 |
| ketone | Frosted Glass in plastic patch with water | Near | 0.7079 |
| ketone | Frosted Glass with transparent post-it sticker | Near | 0.8902 |

| | | | |
|---|---|---|---|
| ¹ Focus indicates the position of the container relative to the optical head. ² "Miss" indicates a failed detection of the analyte. | | | |

| **TABLE 1** | | | |
|---|---|---|---|
| **Analyte** | **Container** | **Focus¹** | **Quality²** |
| ketone | Frosted Glass with translucent post-it sticker | Near | 0.2 |
| ketone | Frosted Glass with transparent scotch tape | Near | 0.5487 |
| ketone | Frosted Glass with transparent scotch tape | Near | 0.3912 |
| ketone | Frosted Glass with transparent scotch tape | near | 0.8867 |

The presence of the surface layer significantly increased the ability to detect ketone, as can be seen from Table 1. When the analyte was in bare frosted glass, the analyte could not be detected in five of eight attempts, a failure rate of nearly 60%. On the other hand, use of a surface layer enabled the analyte to be detected 100% of the time. Moreover, the signal strength was significantly higher when a surface layer was used. Thus, the results demonstrate that the use of a surface layer can be used to improve the detection of analyte.

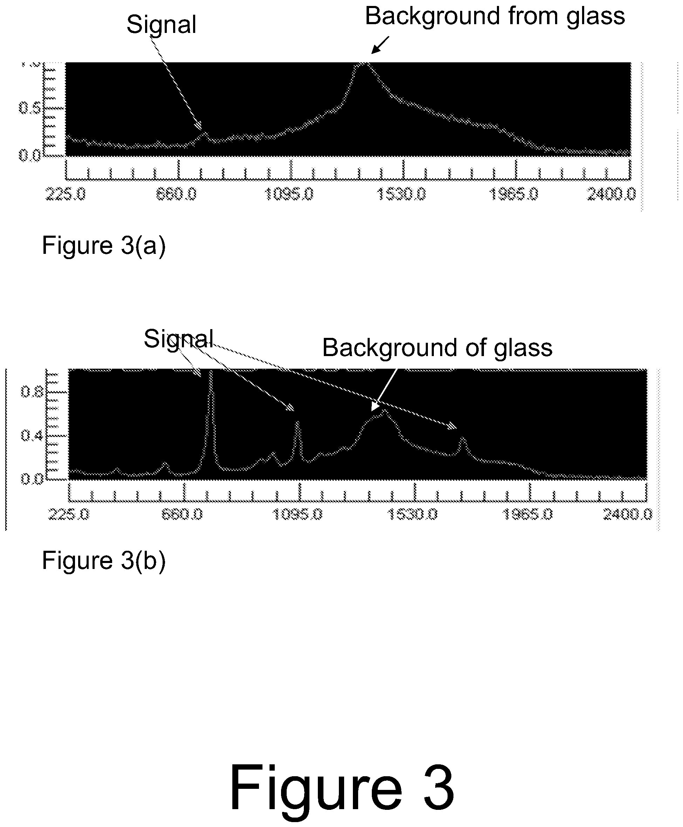

Figure 3 provides an illustration of the signal increase obtained by using a surface layer. Figure 3(a) shows a Raman spectrum obtained without using a surface layer, and Figure 3(b) shows the spectrum when a surface layer in the form of transparent adhesive tape was used. The surface layer greatly increased the strength of the signal from the analyte, a ketone.

The embodiments described above have been set forth herein for the purpose of illustration.

## Claims

1. A method of reducing the loss of electromagnetic radiation during Raman spectroscopy wherein the loss is caused by passing the electromagnetic radiation through material comprising glass or plastic with a rough surface, the method comprising applying a surface layer to the rough surface wherein the material is in the form of a container before electromagnetic radiation is passed through the material, wherein the surface layer decreases the loss of electromagnetic radiation resulting from passing the electromagnetic radiation through the material, and
passing electromagnetic radiation through the container with the surface layer in order to identify an analyte arranged in the container, wherein the surface layer is selected to have optical properties that do not negatively interfere with analysis of the analyte, the analyte being one of an explosive or a narcotic.

2. The method of claim 1, wherein the surface layer is a polymer film.

3. The method of claim 2, wherein the surface polymer film is a transparent adhesive tape.

4. The method of claim 1, further comprising applying a second surface layer to the rough surface.

5. The method of claim 1, further comprising a protective layer, wherein the protective layer is applied to the surface layer.

6. The method of claim 1, wherein the material with a rough surface comprises a portion of frosted glass or frosted plastic.

7. The method of claim 1, wherein Raman spectroscopy is performed on the analyte in the container, the method further comprising:
illuminating the analyte with monochromatic light by passing monochromatic light through the rough surface of the container; and
detecting Raman scattering caused by the analyte using a detector, wherein the surface layer reduces the loss of electromagnetic energy resulting from (i) passing the monochromatic light through the rough surface to the analyte or (ii) the photons passing through the rough surface to the detector.

8. The method of claim 7, wherein the surface layer is a liquid or a plastic film.

9. The method of claim 8, wherein the plastic film is a transparent adhesive film.

10. The method of claim 7, further comprising applying an adhesive to the rough surface before applying the surface layer to the rough surface.

11. The method of claim 7, wherein the analyte is a liquid, for example a liquid explosive.

12. The method of claim 7, wherein the analyte comprises a peroxide solution.

13. The method of claim 7, further comprising forming a Raman profile based on the detected Raman scattering and comparing the Raman profile with a library of known Raman profiles.

14. The method of claim 1, wherein multiple surface layers are applied to the rough surface, wherein the softest or most deformable surface layer is closest to the rough surface.

15. A method of reducing the loss of electromagnetic radiation caused by passing electromagnetic radiation through material with a rough surface, comprising applying a surface layer to the rough surface of a material in the form of a substrate before electromagnetic radiation is passed through the material, wherein the surface is rough due to pits or gaps caused by at least one of: molding, chemical etching, or sandblasting; wherein the surface layer decreases the loss of electromagnetic radiation resulting from passing the electromagnetic radiation through the material, wherein the substrate is the optical head of an analysis instrument.

## Patentansprüche

1. Verfahren zum Reduzieren des Verlusts an elektromagnetischer Strahlung während der Raman-Spektroskopie, wobei der Verlust durch Leiten der elektromagnetischen Strahlung durch Material verursacht wird, das Glas oder Kunststoff mit einer rauen Oberfläche umfasst, wobei das Verfahren das Aufbringen einer Oberflächenschicht auf die raue Oberfläche umfasst wobei das Material in Form eines Behälters vorliegt, bevor elektromagnetische Strahlung durch das Material geleitet wird, wobei die Oberflächenschicht den Verlust an elektromagnetischer Strahlung verringert, der sich aus dem Durchtritt der elektromagnetischen Strahlung durch das Material ergibt, und
Leiten elektromagnetischer Strahlung durch den Behälter, wobei die Oberflächenschicht dafür angeordnet ist, einen Analyten zu identifizieren, der sich in dem Behälter befindet, wobei die Oberflächenschicht dafür ausgewählt ist, optische Eigenschaften aufzuweisen, die die Analyse des Analyten nicht negativ beeinflussen, wobei der Analyt ein Sprengstoff oder ein Narkotikum ist.

2. Verfahren nach Anspruch 1, wobei die Oberflächenschicht eine Polymerfolie ist.

3. Verfahren nach Anspruch 2, wobei der Oberflächenpolymerfilm ein transparentes Klebeband ist.

4. Verfahren nach Anspruch 1, das ferner das Aufbringen einer zweiten Oberflächenschicht auf die raue Oberfläche umfasst.

5. Verfahren nach Anspruch 1, das ferner eine Schutzschicht umfasst, wobei die Schutzschicht auf die Oberflächenschicht aufgebracht wird.

6. Verfahren nach Anspruch 1, wobei das Material mit einer rauen Oberfläche einen Teil aus Mattglas oder Mattkunststoff umfasst.

7. Verfahren nach Anspruch 1, wobei eine Raman-Spektroskopie an dem Analyten in dem Behälter durchgeführt wird, wobei das Verfahren ferner Folgendes umfasst:
Beleuchten des Analyten mit monochromatischem Licht durch Leiten von monochromatischem Licht durch die raue Oberfläche des Behälters; und
Erfassen der durch den Analyten verursachten Raman-Streuung unter Verwendung eines Detektors, wobei die Oberflächenschicht den Verlust an elektromagnetischer Energie reduziert, der sich aus Folgendem ergibt: (i) dem Leiten des monochromatischen Lichts durch die raue Oberfläche zum Analyten oder (ii) dem Leiten von Photonen durch die raue Oberfläche zum Detektor.

8. Verfahren nach Anspruch 7, wobei die Oberflächenschicht eine Flüssigkeit oder eine Kunststofffolie ist.

9. Verfahren nach Anspruch 8, wobei die Kunststofffolie eine transparente Klebefolie ist.

10. Verfahren nach Anspruch 7, das ferner das Aufbringen eines Klebstoffs auf die raue Oberfläche vor dem Aufbringen der Oberflächenschicht auf die raue Oberfläche umfasst.

11. Verfahren nach Anspruch 7, wobei der Analyt eine Flüssigkeit ist, beispielsweise ein flüssiger Sprengstoff.

12. Verfahren nach Anspruch 7, wobei der Analyt eine Peroxidlösung umfasst.

13. Verfahren nach Anspruch 7, das ferner das Bilden eines Raman-Profils basierend auf der erfassten Raman-Streuung und das Vergleichen des Raman-Profils mit einer Bibliothek bekannter Raman-Profile umfasst.

14. Verfahren nach Anspruch 1, wobei mehrere Oberflächenschichten auf die raue Oberfläche aufgebracht werden, wobei die weichste oder am meisten deformierbare Oberflächenschicht der rauen Oberfläche am nächsten liegt.

15. Verfahren zur Verringerung des Verlusts an elektromagnetischer Strahlung, der dadurch verursacht wird, dass elektromagnetische Strahlung durch Material mit rauer Oberfläche geleitet wird, umfassend das Aufbringen einer Oberflächenschicht auf die raue Oberfläche eines Materials in Form eines Substrats, bevor elektromagnetische Strahlung durch das Material geleitet wird, wobei die Oberfläche aufgrund von Löchern oder Spalten rau ist, die durch mindestens eines der Folgenden verursacht werden: Formen, chemisches Ätzen oder Sandstrahlen; wobei die Oberflächenschicht den Verlust an elektromagnetischer Strahlung verringert, der sich aus dem Durchtritt der elektromagnetischen Strahlung durch das Material ergibt, wobei das Substrat der optische Kopfteil eines Analyseinstruments ist.

## Revendications

1. Procédé de réduction de la perte de rayonnement électromagnétique au cours d'une spectroscopie Raman, dans lequel la perte est provoquée par le passage du rayonnement électromagnétique à travers un matériau comprenant du verre ou du plastique avec une surface rugueuse, le procédé comprenant l'application d'une couche de surface sur la surface rugueuse, dans lequel le matériau se présente sous la sous forme d'un récipient, avant que l'on fasse passer le rayonnement électromagnétique à travers le matériau, dans lequel la couche de surface réduit la perte de rayonnement électromagnétique résultant du passage du rayonnement électromagnétique à travers le matériau, et
le passage du rayonnement électromagnétique à travers le récipient avec la couche de surface, afin d'identifier un analyte agencé dans le récipient, dans lequel la couche de surface est choisie pour avoir des propriétés optiques qui n'interfèrent pas de manière négative avec l'analyse de l'analyte, l'analyte étant l'un d'un explosif ou d'un stupéfiant.

2. Procédé selon la revendication 1, dans lequel la couche de surface est un film polymère.

3. Procédé selon la revendication 2, dans lequel le film polymère de surface est un ruban adhésif transparent.

4. Procédé selon la revendication 1, comprenant en outre l'application d'une deuxième couche de surface sur la surface rugueuse.

5. Procédé selon la revendication 1, comprenant en outre une couche protectrice, dans lequel la couche protectrice est appliquée sur la couche de surface.

6. Procédé selon la revendication 1, dans lequel le matériau avec une surface rugueuse comprend une partie de verre dépoli ou de plastique dépoli.

7. Procédé selon la revendication 1, dans lequel la spectroscopie Raman est effectuée sur l'analyte dans le récipient, le procédé comprenant en outre de :
éclairer l'analyte avec une lumière monochromatique en faisant passer une lumière monochromatique à travers la surface rugueuse du récipient; et
détecter la diffusion Raman provoquée par l'analyte en utilisant un détecteur, dans lequel la couche de surface réduit la perte d'énergie électromagnétique résultant (i) du passage de la lumière monochromatique à travers la surface rugueuse vers l'analyte ou (ii) des photons passant à travers la surface rugueuse vers le détecteur.

8. Procédé selon la revendication 7, dans lequel la couche de surface est un liquide ou un film plastique.

9. Procédé selon la revendication 8, dans lequel le film plastique est un film adhésif transparent.

10. Procédé selon la revendication 7, comprenant en outre l'application d'un adhésif sur la surface rugueuse avant d'appliquer la couche de surface sur la surface rugueuse.

11. Procédé selon la revendication 7, dans lequel l'analyte est un liquide, par exemple un explosif liquide.

12. Procédé selon la revendication 7, dans lequel l'analyte comprend une solution de peroxyde.

13. Procédé selon la revendication 7, comprenant en outre la formation d'un profil Raman en fonction de la diffusion Raman détectée et la comparaison du profil Raman avec une bibliothèque de profils Raman connus.

14. Procédé selon la revendication 1, dans lequel de multiples couches de surface sont appliquées sur la surface rugueuse, dans lequel la couche de surface la plus souple ou la plus déformable est la plus proche de la surface rugueuse.

15. Procédé de réduction de la perte de rayonnement électromagnétique provoquée par le passage d'un rayonnement électromagnétique à travers un matériau avec une surface rugueuse, comprenant l'application d'une couche de surface sur la surface rugueuse d'un matériau sous la forme d'un substrat avant que le rayonnement électromagnétique passe à travers le matériau, dans lequel la surface est rugueuse en raison de creux ou de trous provoqués par au moins l'un : d'un moulage, d'une gravure chimique ou d'un sablage; dans lequel la couche de surface réduit la perte de rayonnement électromagnétique résultant du passage du rayonnement électromagnétique à travers le matériau, dans lequel le substrat est la tête optique d'un instrument d'analyse.
